# EUROPÄISCHE PATENTSCHRIFT

(11) **EP 1 023 024 B1**
(45) Veröffentlichungstag und Bekanntmachung des Hinweises auf die Patenterteilung: **10.04.2002**
(21) Anmeldenummer: 98952656.1
(22) Anmeldetag: 29.09.1998
(51) Int. Cl.: A61F 13/42

(54) **WINDEL MIT NÄSSEINDIKATOR**
DIAPER WITH WETNESS INDICATOR
COUCHE MUNIE D'UN INDICATEUR D'HUMIDITE

(30) Priorität: 17.10.1997 DE 19745878
(43) Veröffentlichungstag der Anmeldung: 02.08.2000
(73) Patentinhaber: Paul Hartmann Aktiengesellschaft, 89522 Heidenheim (DE)
(72) Erfinder: MALOWANIEC, Krzysztof, D., D-89522 Heidenheim (DE)
(74) Vertreter: Dreiss, Fuhlendorf, Steimle & Becker
(86) Internationale Anmeldenummer: EP9806157
(87) Internationale Veröffentlichungsnummer: WO9920216

(56) Entgegenhaltungen:
- WO-A-97/10789
- DE-U- 9 309 199
- FR-A- 2 575 905
- US-A- 4 333 463
- US-A- 5 330 456

## Beschreibung

Die Erfindung betrifft einen Hygieneartikel, insbesondere eine Windel, zum einmaligen Gebrauch, mit einem Rückenbereich und einem Bauchbereich, mit einem zumindest abschnittsweise flüssigkeitsdichten Rückenblatt, einem zumindest abschnittsweise flüssigkeitsdurchlässigen Deckblatt und einem dazwischen angeordneten, superabsorbierende Polymere enthaltenden Saugkörper und mit einem Nässeanzeigemittel.

Es sind bereits Hygieneartikel bekannt, die Nässeindikatoren aufweisen, welche ohne dass ein Öffnen der Windel erforderlich ist, Aufschluss darüber geben sollen, ob ein Windelwechsel aufgrund erfolgter Einnässung erforderlich ist oder nicht.

So offenbart die US PS 4,022,211 eine Wegwerfwindel, bei der zwischen Saugkörpermaterial und Rückenblatt eine absorbierende Schicht mit im trockenen Zustand farbigen aber wasserauswaschbaren Motiven positioniert ist.

Die US-PS 4,231,370 zeigt eine Windel, bei der das Rückenblatt innen eine Beschichtung mit einem pH-sensiblen Farbindikator aufweist.

Aus der DE-OS 20 31 104 ist eine Windel bekannt, die an der Außenseite des vorderen Windelrandes mehrere nebeneinander liegende, verfärbbare Indikatoren aufweist, die über jeweils ein streifenförmiges Element aus flüssigkeitsleitendem Werkstoff mit dem Nässungsbereich verbunden sind.

Die WO 95/00099 zeigt auf der Innenseite des Rückenblattes unterhalb des Saugkörpers aufgebrachte Nässeanzeigemittel, die bei Einnässung einen Farbumschlag zeigen oder auswaschbar sind.

Wie in der EP 0 211 524 A1 offenbart, kann das Nässeanzeigemittel auch eine per Tintenstrahldruckverfahren aufgebrachte auswaschbare Substanz sein. Dieses Verfahren eignet sich im Besonderen zur Integration in schnell laufenden Windelherstellungmaschinen und zur Aufbringung der Nässeanzeigemittel in Form von Schriftzügen oder Symbolen.

Aus der US-Patentschrift 5,401,267 ist ein absorbierender Artikel bekannt, bei dem eine obere absorbierende Schicht mit gegenüber der darunterliegenden Schicht höherem Flüssigkeitsleitvermögen vorgesehen ist, wobei die Breite dieser oberen Schicht geringer ist als diejenige der darunterliegenden. Diese obere Schicht vermittelt ein visuelles Signal im Gebrauch des Artikels, welches dem Benutzer signalisiert, dass die aufgenommene Körperflüssigkeit noch in einem Zentralbereich des Artikels absorbiert ist.

Aus der US-Patentschrift 5,354,289 ist ein Hygieneartikel bekannt, bei dem das Nässeanzeigemittel unterhalb eines superabsorbierende Materialien enthaltenden Saugkörpers zwischen der flüssigkeitsundurchlässigen Rückschicht und einer Abdeckschicht vorgesehen ist. In den Saugkörper eingeleitete Flüssigkeit kann zwischen der Rückschicht und der Abdeckschicht zu dem Nässeanzeigemittel gelangen.

Bei einem Hygieneartikel gemäß WO 97/10789 ist ein Nässeanzeigemittel entweder unmittelbar angrenzend an eine Flüssigkeitsspeicher- und Verteilerschicht, welche superabsorbierende Materialien enthalten kann, oder unterhalb dieser angeordnet.

Aus der inzwischen vollzogenen Weiterentwicklung des Saugkörpermaterials resultiert ein Problem: Früher wurde praktisch ausschließlich auf technische Cellulose-Fasern als Saugkörpermaterial zurückgegriffen, sei es in Papier oder papierähnlicher Form oder in Form trocken zerfaserten Zellstoffes. Dieses Material nimmt wässrige Flüssigkeiten sofort auf, so dass davon ausgegangen werden kann, dass erst bei Erschöpfung der Saugkapazität der Cellulosefasern Flüssigkeit den unterhalb des Saugmaterials positionierten Nässeindikator erreicht.

Heute werden aber in zunehmend hohem Anteil neben den Cellulose-Fasern sogenannte superabsorbierende Polymere (SAP) eingesetzt, die ein Vielfaches ihres Eigengewichtes an wässrigen Flüssigkeiten (z. B. Urin) durch Quellung binden können. Nun erfolgt diese Quellung aber nicht spontan, sondern mit erheblichem Zeitverzug, der im Windelverbund im Bereich von etwa 30 min liegen kann. Es kann daher vorkommen, dass anfallende Flüssigkeit das Saugkörpermaterial nach den Gesetzen der Schwerkraft zunächst in vertikaler Richtung passiert, das Rückenblattmaterial erreicht und den dort vorhandenen Nässeindikator verfärbt, auswäscht o. ä. und erst anschließend vom superabsorbierenden Polymer des Saugkörpers absorbiert wird. Es würde also ein Windelwechsel als erforderlich angezeigt werden, obwohl die Absorptionskapazität des Saugkörpers noch nicht erschöpft ist.

Der vorliegenden Erfindung liegt deshalb die Aufgabe zugrunde, einen Hygieneartikel mit SAP-enthaltendem Saugkörper und mit Nässeanzeigemitteln zu schaffen, die einen Windelwechsel zuverlässig erst dann signalisieren, wenn dieser hinsichtlich der noch verbleibenden Saugkapazität auch wirklich erforderlich ist.

Die Aufgabe wird gelöst durch einen Hygieneartikel mit den im Anspruch 1 genannten Merkmalen.

Der zentrale SAP-enthaltende Teilbereich des Saugkörpers bestimmt im Wesentlichen die Absorptionskapazität des Hygieneproduktes. Erst wenn diese erschöpft ist, kann zusätzlich anfallende Flüssigkeit in den SAP-freien Bereich vorstoßen und dort das Nässeanzeigemittel aktivieren, so dass der dann sicherlich erforderliche Windelwechsel angezeigt werden kann. Solange wirkt der SAP-freie Bereich quasi als Abschirmung des Nässeanzeigemittels.

Der den zentralen Teilbereich umgebende Bereich des Saugkörpers ist in Richtung seiner gesamten Dicke, also senkrecht zur Ebene der flächenhaften Erstreckung des Saugkörpers SAP-frei.

Gemäß einer bevorzugten Ausführungsform des Hygieneartikels sind die Nässeanzeigemittel zumindest 2 cm, bevorzugt zumindest 3 cm von der Kante des zentralen SAP-enthaltenden Saugkörperteilbereiches, bevorzugt von der Seitenkante des Saugkörperteilbereiches entfernt angebracht. Dies stellt sicher, dass die Absorptionskapazität der SAP-haltigen Schicht auch wirklich erschöpft ist, wenn die Nässeanzeigemittel benässt und damit aktiviert werden.

Gemäß einer weiteren bevorzugten Ausführungsform beträgt das Flächengewicht des SAP-freien Bereiches 50 - 250 g/m², bevorzugt 100- 200 g/m² und besonders bevorzugt 130 - 170 g/m². Es hat sich gezeigt, dass damit gerade genug Absorptionsmaterial im SAP-freien Bereich zur Verfügung steht, um ein zu schnelles Auslaufen der Windel nach Aktivierung der Nässeanzeigemittel zu verhindern, das heißt, dass ausreichend Zeit für den erforderlichen Windelwechsel verbleibt.

Es versteht sich, dass zur Bildung des SAP-freien Bereiches nicht notwendigerweise ein zweischichtiger Saugkörper Verwendung finden muss. Auch ein einschichtiger Saugkörper kann einen zentralen SAP-haltigen Bereich und einen peripheren SAP-freien Bereich aufweisen.

Ebenso kann der Saugkörper mehr als die beschriebenen zwei Schichten aufweisen, z. B. zusätzlich eine über der SAP-enthaltenden Schicht zu liegen kommende Flüssigkeitsaufnahmeund Verteilerschicht auf Vliesstoffbasis und/oder auf der Basis chemisch vernetzter Cellulosefasern.

Weitere Merkmale, Einzelheiten und Vorteile der Erfindung ergeben sich aus der zeichnerischen Darstellung und nachfolgenden Beschreibung einer bevorzugten Ausführungsform des erfindungsgemäßen Hygieneartikels. In der Zeichnung zeigt:
- Figur 1: eine Draufsicht auf eine Windel; und
- Figur 2: eine Schnittansicht in Richtung der Pfeile II-II in Figur 1.

Eine Wegwerfwindel 2 weist einen Bauchbereich 4 und einen Rückenbereich 6 auf. Am Rückenbereich 6 sind Verschlussstreifen 8 angebracht, mit deren Hilfe die Wegwerfwindel 2 in überlappender Konfiguration am Körper fixiert werden kann. Die Wegwerfwindel 2 umfasst ein flüssigkeitsdurchlässiges Deckblatt 10, z. B. ein Vlies oder eine perforierte Folie oder auch ein entsprechendes Laminat, ein flüssigkeitsdichtes Rückenblatt 12, z. B. eine dünne Kunststofffolie oder ein Vlieslaminat oder ein Vlies/Folienlaminat, und einen Saugkörper 14.

Der Saugkörper 14 ist im vorliegenden Fall zweischichtig ausgeführt. Die obere körperseitige Schicht 16 ist eine superabsorbierende Polymere (SAP) enthaltende Schicht, die neben SAP auch Fasermaterial, bevorzugt cellulosisches Fasermaterial enthält. Der SAP-Anteil trägt bevorzugt 5 - 75 Gew.-%. Die untere Schicht 18 ist SAP-frei und besteht im Wesentlichen ausschließlich aus Fasermaterial, bevorzugt aus Cellulosefasern. Die Projektionsfläche der unteren Schicht 18 senkrecht zur Ebene der flächenhaften Erstreckung des Saugkörpers bzw. der Windel betrachtet, ist größer als die der oberen Schicht 16, welche vollständig innerhalb der Projektionsfläche der unteren Schicht 18 zu liegen kommt.

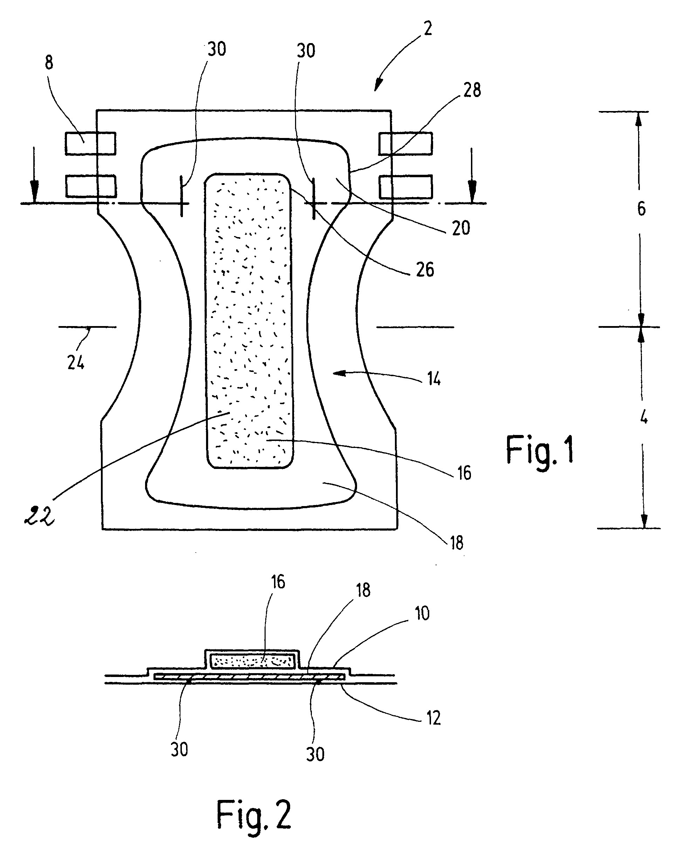

Damit ist ein äußerer, über die gesamte Dicke des Saugkörpers SAP-freier Bereich 20 gebildet, der in diesem Fall einen zentralen SAP-haltigen Teilbereich 22 in Form der oberen Schicht 16 vollständig umgibt. Der im Rückenbereich 6 der Windel parallel zur Windelquerachse 24 gemessene weiteste Abstand zwischen der Seitenkante 26 der oberen Saugkörperschicht 16 und einer Seitenkante 28 der unteren Saugkörperschicht 18 beträgt bevorzugt mindestens 4 cm. Im Rückenbereich 6, direkt auf der Innenseite des Rückenblattes 12 im Bereich 20 sind eine beliebige Anzahl von Nässeanzeigemitteln 30 in einem frei wählbaren Muster angeordnet. Im einfachsten Fall handelt es sich wie in Fig. 1 dargestellt um ein an jeder Seite im Rückenbereich 6 aufgebrachtes streifenförmiges Nässeanzeigemittel 30.

Vorteilhafterweise kann das Nässeanzeigemittel 30 auch in Form eines Schriftzuges ausgeführt sein.

Das Nässeanzeigemittel 30 kann z. B. ein auf Nässe- oder ph-Wechsel ansprechender Farbindikator sein, der in ein Trägermaterial, z. B. einen Schmelzkleber integriert ist. Beispiele sind der US-PS 5 035 691, der US-PS 5 066 711, der US-PS 5 342 861, der US-PS 4 895 567 oder der US-PS 4 231 370 zu entnehmen.

## Patentansprüche

1. Hygieneartikel, insbesondere Windel zum einmaligen Gebrauch, mit einem Rückenbereich (6) und einem Bauchbereich (4), mit einem zumindest abschnittsweise flüssigkeitsdichten Rückenblatt (12), einem zumindest abschnittsweise flüssigkeitsdurchlässigen Deckblatt (10) und einem dazwischen angeordneten, superabsorbierende Polymere enthaltenden Saugkörper (14) und mit einem Nässeanzeigemittel (30), **dadurch gekennzeichnet, dass** die superabsorbierenden Polymere ausschließlich in einem zentralen Teilbereich (16) des Saugkörpers (14) angeordnet sind, der in Projektion senkrecht zur Ebene des Hygieneartikels betrachtet von einem von superabsorbierenden Polymeren freien im Wesentlichen ausschließlich Cellulosefasern enthaltenden Bereich (18) umgeben ist, und dass das Nässeanzeigemittel (30) zwischen dem Rückenblatt (12) und dem von superabsorbierenden Polymeren freien Bereich (18) des Saugkörpers (14) angeordnet ist.

2. Hygieneartikel nach Anspruch 1, **dadurch gekennzeichnet, dass** das Nässeanzeigemittel (30) auf Feuchtigkeit ansprechende Farbindikatoren zusammen mit einem Trägermaterial umfasst.

3. Hygieneartikel nach Anspruch 1, **dadurch gekennzeichnet, dass** Nässeanzeigemittel (30) auf einen pH-Wert-Wechsel ansprechende Farbindikatoren zusammen mit einem Trägermaterial umfasst.

4. Hygieneartikel nach Anspruch 2 oder 3, **dadurch gekennzeichnet, dass** als Trägermaterial ein Schmelzkleber vorgesehen ist.

5. Hygieneartikel nach Anspruch 1, **dadurch gekennzeichnet, dass** das Nässeanzeigemittel (30) auswaschbare farbige Substanzen, inbesondere Tinte umfasst.

6. Hygieneartikel nach einem der vorstehenden Ansprüche, **dadurch gekennzeichnet, dass** das Nässeanzeigemittel (30) im Rückenbereich (6) und/oder im Bauchbereich (4) innenseitig auf dem Rückenblatt (12) angebracht ist.

7. Hygieneartikel nach einem der vorstehenden Ansprüche, **dadurch gekennzeichnet, dass** das Nässeanzeigemittel in Querrichtung (24) des Artikels zumindest 2 cm bevorzugt mindestens 3 cm von einer Kante (26) des SAP-haltigen Bereiches (16) des Saugkörpers (14) entfernt angeordnet ist.

8. Hygieneartikel nach einem der vorstehenden Ansprüche, **dadurch gekennzeichnet, dass** das Flächengewicht des SAP-freien Bereiches (18) des Saugkörpers50 - 250 g/m², bevorzugt 100 - 200 g/m², besonders bevorzugt 130 - 170 g/m² beträgt.

## Claims

1. Sanitary article, in particular disposable napkin, comprising a back region (6) and a front region (4), a back sheet (12) which is liquid-tight at least in sections, a top sheet (10) which is liquid-permeable at least in sections and an absorbent body (14) containing super-absorbent polymers arranged therebetween, and a wetness indicator (30), **characterised in that** the super-absorbent polymers are arranged exclusively in a central partial region (16) of the absorbent body (14) which is surrounded by a region (18) free of super-absorbent polymers and containing essentially exclusively cellulose fibres as viewed perpendicularly to the plane of the sanitary article, and that the wetness indicator (30) is arranged between the back sheet (12) and the region (18) of the absorbent body (14) free of super-absorbent polymers.

2. Sanitary article according to claim 1, **characterised in that** the wetness indicator (30) includes colour indicators responding to moisture together with a substrate.

3. Sanitary article according to claim 1, **characterised in that** the wetness indicator (30) includes colour indicators responding to a change in pH value together with a substrate.

4. Sanitary article according to claim 2 or claim 3, **characterised in that** a hot-melt adhesive is provided as the substrate.

5. Sanitary article according to claim 1, **characterised in that** the wetness indicator (30) includes coloured substances removable by washing, in particular ink.

6. Sanitary article according to one of the preceding claims, **characterised in that** the wetness indicator (30) is applied to the inside of the back sheet (12) in the back region (6) and/or in the front region (4).

7. Sanitary article according to one of the preceding claims, **characterised in that** the wetness indicator is arranged at a distance of at least 2 cm, preferably at least 3 cm from one edge (26) of the SAP-containing region (16) of the absorbent body (14) in the transverse direction (24) of the article.

8. Sanitary article according to one of the preceding claims, **characterised in that** the weight per unit area of the SAP-free region (18) of the absorbent body is 50 - 250 g/m², preferably 100 - 200 g/m², particularly preferably 130 - 170 g/m².

## Revendications

1. Article hygiénique, en particulier couche à usage unique, qui comprend une zone de dos (6) et une zone de ventre (4) et qui comporte une feuille de dos (12), qui est , au moins par sections, étanche aux liquides, une feuille de recouvrement (10), qui est, au moins par sections, étanche aux liquides, un corps absorbant (14), qui est disposé entre les deux feuilles et qui contient des polymères superabsorbants, et un moyen indicateur de l'humidité (30), **caractérisé en ce que** les polymères superabsorbants sont exclusivement disposés dans une zone centrale (16) du corps absorbant (14) qui, vu en projection perpendiculairement au plan de l'article hygiénique, est entouré par une zone (18), qui est libre de polymères superabsorbants et qui contient, pour l'essentiel, exclusivement des fibres de cellulose et **en ce que** le moyen indicateur de l'humidité (30) est disposé entre la feuille de dos (12) et la zone (18) du corps absorbant (14) qui est libre de polymères superabsorbants.

2. Article hygiénique selon la revendication 1, **caractérisé en ce que** le moyen indicateur de l'humidité (30) comprend des indicateurs colorés sensibles à l'humidité et associés à un matériau support.

3. Article hygiénique selon la revendication 1, **caractérisé en ce que** le moyen indicateur de l'humidité (30) comprend des indicateurs colorés sensibles à une variation de la valeur du pH et associés à un matériau support.

4. Article hygiénique selon la revendication 2 ou 3, **caractérisé en ce que**, en tant que matériau support, on prévoit une colle fusible.

5. Article hygiénique selon la revendication 1, **caractérisé en ce que** le moyen indicateur de l'humidité (30) comprend des substances colorées qui peuvent être enlevées par lavage, en particulier de l'encre.

6. Article hygiénique selon l'une quelconque des revendications précédentes, **caractérisé en ce que** le moyen indicateur de l'humidité (30) est appliqué sur la face intérieure sur la feuille de recouvrement (12) dans la zone de dos (6) et/ou dans la zone de ventre (4).

7. Article hygiénique selon l'une quelconque des revendications précédentes, **caractérisé en ce que** le moyen indicateur de l'humidité (30) est disposé en étant éloigné, dans la direction transversale (24) de l'article, d'au moins 2 cm, de préférence 3 cm, d'un bord (26) de la zone (16) du corps absorbant (14) qui contient des polymères superabsorbants.

8. Article hygiénique selon l'une quelconque des revendications précédentes, **caractérisé en ce que** le grammage de la zone (18) libre de polymères superabsorbants du corps absorbant (14) est comprise entre 50 et 250 g/m², de préférence entre 100 et 200 g/m², particulièrement de préférence entre 130 et 170 g/m².
